# EUROPEAN PATENT APPLICATION

(11) **EP 1 297 830 A1**
(43) Date of publication of application: **02.04.2003**
(21) Application number: 01830612.6
(22) Date of filing: 28.09.2001
(51) Int. Cl.: A61K 31/198, A61K 31/4172, A61P 39/06, A61K 38/05, A61K 31/417, C07K 5/02

(54) **Use of alpha- or beta-amino acids, of the corresponding esters or of dipeptides of these amino acids with histidine derivatives in the prevention or treatment of tissue damage caused by a atmospheric ozone**

(71) Applicant: Flamma Fabbrica Lombarda Ammino Acidi S.p.a., 24040 Chignolo d'Isola ( Bergamo) (IT)
(72) Inventor: Bozzetto, Luca, 24100 Bergamo (IT); Genitrini, Paola, 46100 Mantova (IT)
(74) Representative: Long, Giorgio

(57) **Abstract**

Use of at least a compound of formula (I) 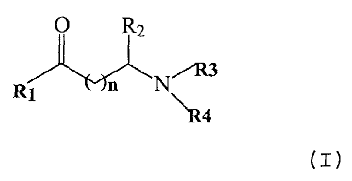 in which n, R₁, R₂, R₃ and R₄ have the meanings given in the description, and the pharmaceutically acceptable salts thereof, for the manufacture of a medicament for preventing or treating tissue damage caused by a high level of atmospheric ozone, and use of the abovementioned compound of formula (I), and cosmetically suitable salts thereof, as an anti-cutaneous-stress cosmetic agent.

## Description

The present invention relates to the use of aminocarbonyl compounds for the manufacture of a medicament for preventing or treating tissue damage caused by a high level of atmospheric ozone.

Throughout the present description and the claims, the expression "a high level of atmospheric ozone" means an external environment in which the amount of ozone is greater than 5 ppb.

It is known that acute and chronic exposure to ozone causes a great deal of tissue damage and adverse cosmetic effects on the skin.

Typical examples of tissue damage are damage to skin tissues and to the tissues of the respiratory tract.

Examples of damage to the tissues of the respiratory tract caused on human health are inflammation which leads to coughing, irritation of the nose and throat, a reduction in pulmonary function which is the cause of emphysema and bronchitis, sensitization to allergens leading, in certain cases, to asthma attacks, and a reduction of the immune system.

Examples of damage to skin tissues are those leading to the formation of spots, dehydration, loss of elasticity, early formation of wrinkles, reddening and loss of radiance.

It has now been found, surprisingly, that aminocarbonyl compounds of formula (I) 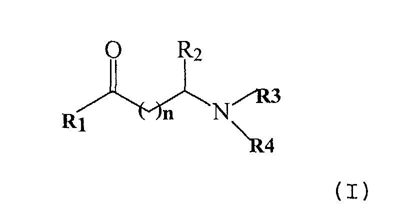 are capable of preventing or treating tissue damage caused by high levels of atmospheric ozone.

A first object of the present invention is thus the use of at least a compound of formula (I) 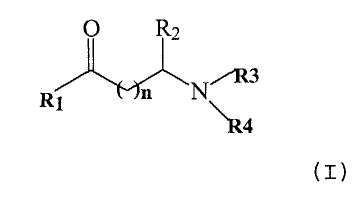 in which
n is 0 or 1,
R₁ is -OR₅ in which R₅ is hydrogen, linear or branched C1-C10 alkyl, C3-C10 cycloalkyl, C6-C10 aryl and C7-C10 arylalkyl or -NH- (CHR₆)-CH₂-(5-imidazole) in which R₆ is hydrogen or -COOR₅ in which R₅ has the meanings given above,
R₂ is chosen from the group comprising hydrogen, linear or branched C1-C10 alkyl, a group -R₇X in which R₇ is linear or branched, saturated or unsaturated C1-C18 alkyl, and X is a group chosen from imidazole, -N(R₈R'₈), -SR₈ in which R₈ and R'₈, which may be identical or different, are chosen from hydrogen, linear or branched C1-20 alkyl or alkenyl, C6-C10 aryl and C7-C10 arylalkyl; R₃ and R₄, which may be identical or different, are chosen from H, linear or branched C1-C10 alkyl, C3-C10 cycloalkyl, C6-C10 aryl, C7-C10 arylalkyl and alkylcarbonyl in which the C1-C20 alkyl is linear or branched and saturated or unsaturated; and
pharmaceutically acceptable salts thereof,
for the manufacture of a medicament for preventing or treating tissue damage caused by high levels of atmospheric ozone.

Preferably, the said tissue damage is chosen from damage to skin tissues and to the tissues of the respiratory tract. Even more preferably, the damage to the tissues of the respiratory tract is an inflammation which leads to coughing, irritation of the nose and throat, a reduction in pulmonary function which is the cause of emphysema or bronchitis, sensitization to allergens leading to asthma attacks and reduction in the immune system, and the skin tissue damage is that leading to the formation of spots, dehydration, loss of elasticity, formation of wrinkles, reddening and loss of radiance.

Preferably, R₁ is chosen from -OH and -(CHR₆)-CH₂-(5-imidazole) in which R₆ is H, -COOH or -COOR₅ in which R₅ is chosen from methyl, ethyl, tert-butyl and octyl.

Preferably, R₂ is chosen from the group comprising hydrogen, linear or branched C1-C6 alkyl, a group -R₇X in which R₇ is linear or branched, saturated or unsaturated C1-C10 alkyl, and X is a group chosen from imidazole, -N(R₈R'₈), -SR₈ in which R₈ and R'₈, which may be identical or different, are chosen from hydrogen, linear or branched C1-C10 alkyl or alkenyl, phenyl and benzyl.

Preferably, R₃ and R₄, which are different, are chosen from H, linear or branched C1-C4 alkyl, C5-C6 cycloalkyl, phenyl, benzyl and alkylcarbonyl in which the C1-C18 alkyl is linear or branched and saturated or unsaturated.

Preferred examples of compounds of the formula (I) are:

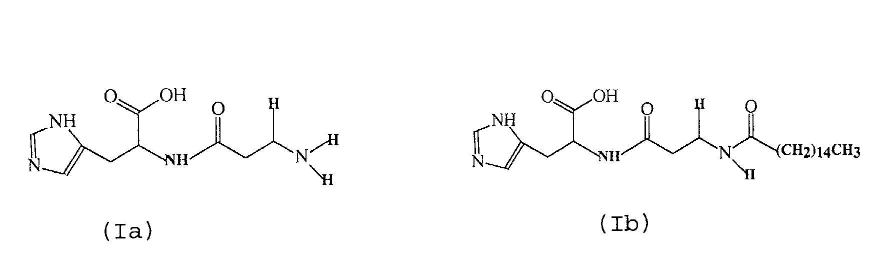

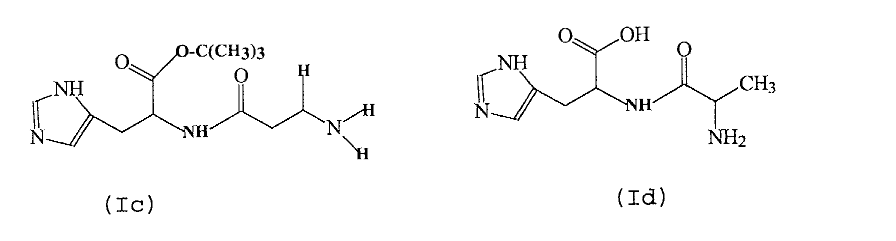

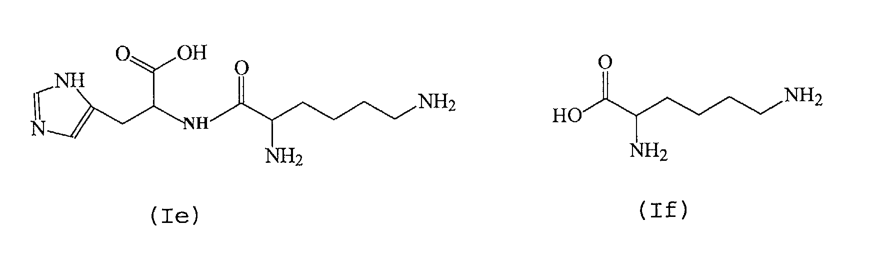

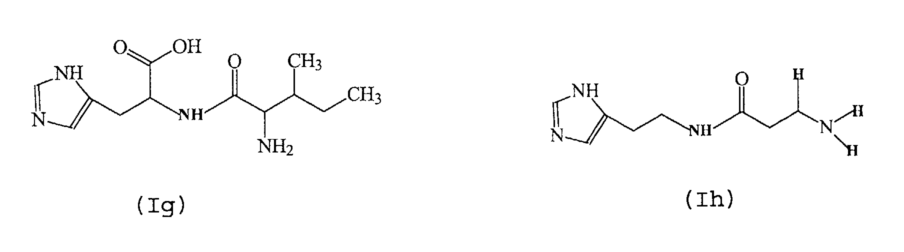

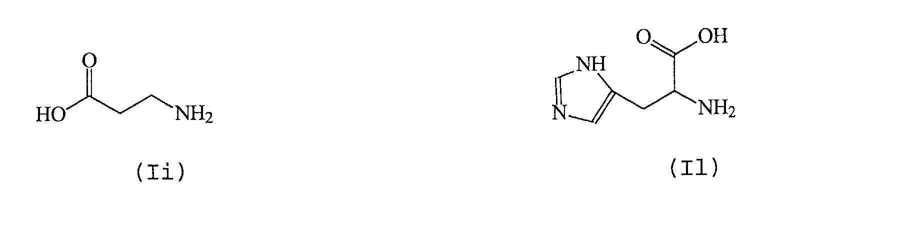

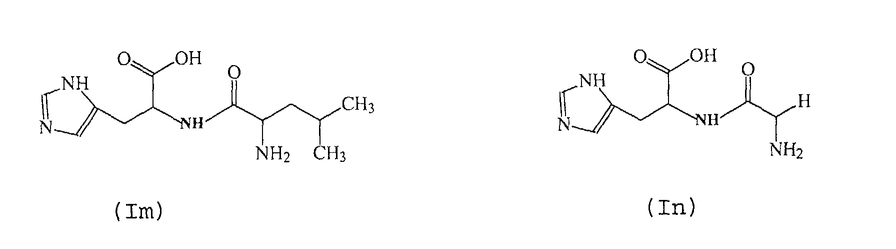

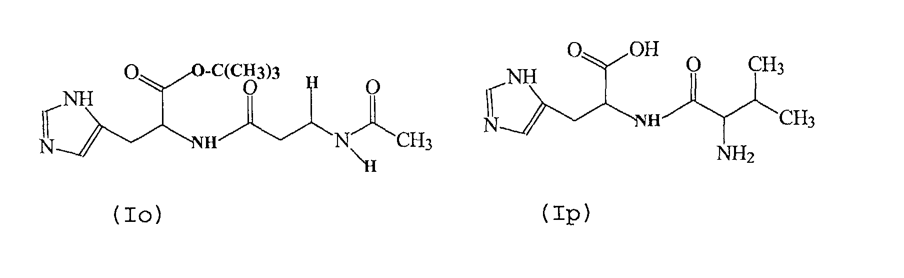

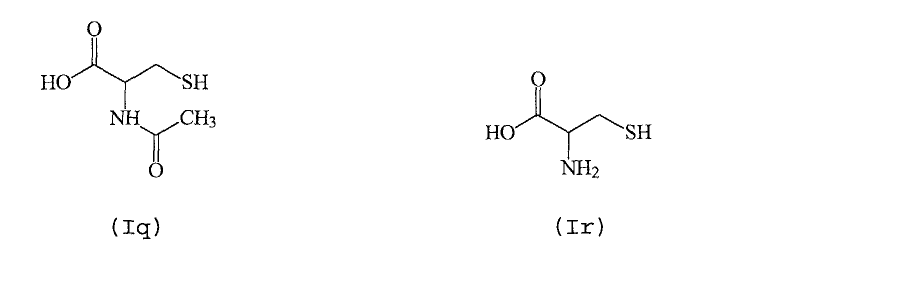

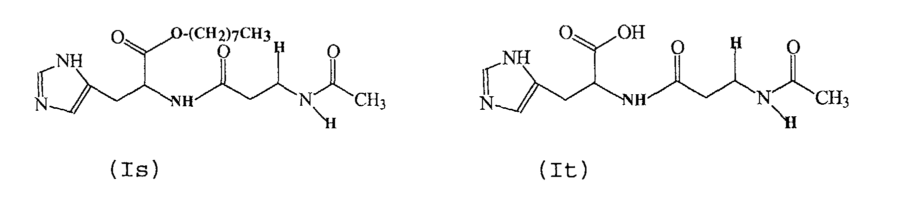

Throughout the present description and the claims, the abovementioned compounds, from left to right, are carnosine (compound of formula Ia), palmitoylcarnosinamide (compound of formula Ib), carnosine tert-butyl ester (compound of formula Ic), the dipeptide Ala-His (compound of formula Id), the dipeptide Lys-His (compound of formula Ie), lysine (compound of formula If), the dipeptide Ile-His (compound of formula Ig), decarboxycarnosine (compound of formula Ih), β-alanine (compound of formula Ii), histidine (compound of formula I1), the dipeptide Leu-His (compound of formula Im), the dipeptide Gly-His (compound of formula In), N-acetylcarnosine tert-butyl ester (compound of formula Io), the dipeptide Val-His (compound of formula Ip), N-acetylcysteine (compound of formula Iq), cysteine (compound of formula Ir), N-acetylcarnosine octyl ester (compound of formula Is) and N-acetylcarnosine (compound of formula It).

A second object of the present invention thus consists of the use of the aminocarbonyl compounds of formula (I) of the present invention, and the salts thereof, as anti-cutaneous-stress cosmetic agents, in particular non-oxidative anti-cutaneous-stress.

Specifically, the Applicant has found that when the aminocarbonyl compounds of formula (I) of the present invention and the salts thereof are subjected directly to a high level of atmospheric ozone, they do not react with the ozone. However, when the abovementioned experiment is repeated *in vivo* by treating the skin of mice with compounds of the present invention and then exposing it to ozone, it was shown that the compounds of the present invention are capable of preventing non-oxidative cutaneous stress, that is to say cutaneous stress not linked to the oxidative properties of ozone. In other words, the compounds of the present invention do not behave *in vivo* as ozone scavengers.

The aminocarbonyl compounds of formula (I) of the present invention except for carnosine (compound of formula Ia), palmitoylcarnosinamide (compound of formula Ib), the dipeptide Ala-His (compound of formula Id), the dipeptide Lys-His (compound of formula Ie), the dipeptide Ile-His (compound of formula Ig), decarboxycarnosine (compound of formula Ih), β-alanine (compound of formula Ii), the dipeptide Leu-His (compound of formula Im), the dipeptide Gly-His (compound of formula In), the dipeptide Val-His (compound of formula Ip), N-acetylcarnosine (compound of formula It) and when, simultaneously, R₁ is OR₅, in which R₅ is hydrogen, and n is 0, are novel.

These compounds therefore constitute a third object of the present invention.

Preferred examples of compounds of formula (I) of the present invention are those chosen from carnosine tert-butyl ester (compound of formula Ic), N-acetylcarnosine tert-butyl ester (compound of formula Io) and N-acetylcarnosine octyl ester (compound of formula Is).

For practical purposes, the compounds of formula (I) of the present invention and the pharmaceutically acceptable or cosmetically suitable salts thereof may be administered as such, but are preferably administered in the form of pharmaceutical or cosmetic compositions.

The pharmaceutical or cosmetic compositions of the present invention may be in the form, for example, of a tablet, a sugar-coated tablet, a capsule and delayed-release forms, a suppository, a cream, a gel with various degrees of viscosity, an ointment, a solution, a suspension, an emulsion of oil-in-water (O/W) or water-in-oil (W/O) type, a balm, an aerosol, a spray or eye drops.

Preferably, when the said compositions are of pharmaceutical type, they are in the form of a cream, a nasal spray, a tablet, a capsule, an aerosol or eye drops, whereas when they are of cosmetic type they are preferably in the form of an emulsion, a balm, eye drops or a cream.

In addition to the usual excipients, the said pharmaceutical or cosmetic compositions of the present invention may contain additives that are suitable for pharmaceutical or cosmetic use such as, for example, preserving agents, stabilizers, salts for regulating the osmotic pressure, buffers, flavourings, vitamins, antioxidants, wetting agents, bactericides, fragrances, fatty substances, waxes, oils and electrolytes.

Typical examples of suitable vitamins are vitamin A, B15, C, D3 and E, and even more typically, the vitamin is vitamin E.

Typical examples of suitable antioxidants are those based on polyphenols, such as procyanidine derivatives, extract of pomegranate and extract of green tea.

Preferably, the said antioxidants are extract of pomegranate and extract of green tea.

The extract of pomegranate used in the present invention is that obtained from the seeds of *Punica granatum* according to the conventional techniques of maceration, percolation and extraction in a suitable solvent such as, for example, ethanol at a temperature of between 60 and 70°C.

The extract of green tea used in the present invention is that obtained from the leaves of *Camelia sinensis* according to the conventional techniques of extraction in a suitable solvent such as, for example, an ethanol/water or water/ketone mixture in which the weight ratio between the leaves/solvent is 1/1 or 1/5, at a temperature of between 18 and 25°C. La aqueous-alcoholic fraction obtained is then back-extracted with suitable solvents capable of removing unwanted substances such as, for example, chlorophyl, terpenes and caffeine. The aqueous phase collected is then extracted with suitable solvents, discarded and the organic phase is finally evaporated to dryness to give an extract in the form of powder.

If particular treatments require it, the pharmaceutical compositions of the present invention may contain other compatible active ingredients whose simultaneous administration is therapeutically useful.

For practical purposes, the effective amount of the compounds of formula (I) of the present invention which needs to be administered may vary within a relatively broad range depending on known factors such as the specific treatment required, the pharmaceutical or cosmetic composition, the route of administration and the efficacy of the specific compound of the present invention used. However, the optimum effective amount may readily be determined by routine procedures.

The pharmaceutical or cosmetic compositions of the present invention can be produced according to the conventional techniques of the pharmaceutical chemist including mixing, granulation and tabletting, when necessary, or various mixings and dissolutions of the ingredients as is most appropriate to obtain the desired product.

The compounds of formula (I) of the present invention comprise carnosine and derivatives thereof, dipeptides or individual amino acids of natural or synthetic origin. These are therefore prepared according to methods known to those skilled in the art. For example, when the compounds of formula (I) of the present invention are dipeptides, they can be prepared according to the method described in Pinelli C. et al., *Il Farmaco,* Ed. Sc., vol. 23, volume 9, 859-869, entitled "Sintesi di peptidi della β-alanina" [Synthesis of peptides of β-alanine] in which the β-alanine, protected beforehand at the amine group by means of a carbobenzoxy group, is condensed with an amino acid which is free or protected at the carboxyl group by formation of the corresponding methyl or ethyl ester or the mixed anhydride, followed by removal of the protecting groups.

The examples which follow serve to illustrate the present invention without, however, limiting it in any way.

### Preparation of carnosine (compound of formula Ia)

### a) Preparation of Z-β-ala-L-His-OMe

Z-β-ala-Onp (2.76 g, 0.008 mol) and triethylamine (2.17 g, 0.021 mol) were added, with stirring, to a solution of L-His-OMe (1.92 g, 0.008 mol) in pyridine (50 ml). After stirring for 1 hour and leaving to stand at room temperature overnight, the solution was evaporated to dryness under vacuum. The residue obtained was dissolved in ethyl acetate (150 ml) and the solution, washed with Na₂CO₃ (5%) and water, was dried over Na₂SO₄ and then evaporated to dryness.

The desired product was obtained in a yield of 89% after crystallization from ethanol/petroleum ether and had the following physicochemical properties: m.p. 103-104°; Rf₂ = 0.65 and [α]²²_{D} = - 0.9° (1% in CH₃OH) .

### b) Preparation of Z-β-ala-L-His

The product obtained in the abovementioned step a) was saponified according to the method described by Davis and Smith in *Bioch. Preparat.,* 4, 38, 1955.

The desired product was obtained in a yield of 80% and had the following physicochemical properties: m.p. 166-167° and [α]²²_{D} = - 16.6° (1% in H₂O).

### c) Preparation of β-ala-L-His (carnosine)

The product obtained in the abovementioned step b) was subjected to catalytic hydrogenolysis according to the method described in *Bioch. Preparat.,* 4, 38, 1955.

The desired product was obtained in a yield of 85% and had the following physicochemical properties: m.p. 260-261°; Rf₂ = 0.07 and [α]²²_{D} = - 21.3° (2% in H₂O).

### Examples of cosmetic compositions

| Example 1 | |
|---|---|
| Moisturizing O/W fluid emulsion | |
| Components | % (w/w) |
| Glyceryl stearate, PEG-100 stearate | 2.25 |
| Cetearyl alcohol, Ceteareth-20 | 2.00 |
| Squalene | 3.00 |
| Cocoglyceride | 3.00 |
| Tocopheryl acetate | 1.00 |
| Extract of pomegranate | 2.00 |
| L-Carnosine | 1.00 |
| Proline, leucine, arginine aspartate | 2.00 |
| Water, preserving agents | q.s. 100 |
| Glyceryl polyacrylate | 5.00 |
| Glycerol | 9.00 |
| Glucose, carrageenan, glucuronic acid | 4.00 |
| Saccharide isomerate | 1.00 |
| Fragrance | 0.25 |

| Example 2 | |
|---|---|
| Aftersun O/W hyperfluid emulsion | |
| Components | % (w/w) |
| Glyceryl stearate, PEG-100 stearate, Cetearyl alcohol, Ceteareth-12, Cetyl palmitato | 4.25 |
| Ceteareth-20 | 1.00 |
| Cococaprylate/caprate | 5.00 |
| Dicapryl ether | 5.00 |
| Panthenol | 1.00 |
| Bisabolol | 1.00 |
| Extract of pomegranate | 2.00 |
| Tocopheryl acetate | 1.00 |
| L-Carnosine | 1.00 |
| Proline, N-acetylcystein, leucine, arginine aspartate | 2.00 |
| Water, preserving agents | q.s. 100 |

| Example 3 | |
|---|---|
| Hair conditioning balm | |
| Components | % (w/w) |
| Dicetearylethyl dimonium chloride | 2.00 |
| Ceteareth-3 | 1.00 |
| Cetyl alcohol | 1.00 |
| Extract of pomegranate | 2.00 |
| L-Carnosine | 0.50 |
| N-Acetylcystein | 1.00 |
| Proline, leucine, arginine aspartate | 2.00 |
| Water, preserving agents | q.s. 100 |
| Panthenol | 0.50 |
| Citric acid | q.s. pH 3.5 |
| Fragrance | 0.25 |

### Examples of pharmaceutical compositions

| Example 1 | |
|---|---|
| Capsules | |
| Components | Mg |
| Carnosine | 400 |
| Sodium carboxymethylstarch | 10 |
| Talc | 81 |
| Magnesium stearate | 13 |
| Corn starch | 36 |
| Gelatin | 95.05 |
| E 171 | 1.95 |

| Example 2 | |
|---|---|
| Tablets | |
| Components | Mg |
| Carnosine | 500 |
| Corn starch | 30 |
| Microcrystalline cellulose | 13 |
| Hydroxypropylcellulose | 12 |
| Saccharine | 10 |
| Magnesium stearate | 10 |
| Flavouring | 10 |

| Example 3 | |
|---|---|
| Nasal spray | |
| Components | Mg |
| Carnosine | 5 |
| Menthol | 15 |
| Essence of mint | 1.4 |
| Isopropyl myristate | 239 |
| Lanolin | 30 |
| Sodium chloride | 18 |
| Trichlorotrifluoroethane | 2.958 |
| Dichlorodifluoromethane | 2.208 |
| Dichlorotetrafluoroethane | 8.833 |

| Example 4 | |
|---|---|
| Aerosol | |
| Components | Mg |
| Carnosine | 10 |
| Oleic acid | 1 |
| Dichlorodifluoromethane | 12.2 |
| Trichlorofluoromethane | 4.7 |

| Example 5 | |
|---|---|
| Eye drops | |
| Components | Mg |
| Carnosine | 1 |
| Benzalkonium chloride | 15 |
| Boric acid | 1.4 |
| Borax | 239 |
| Injection-grade water | q.s. 1 ml |

### Test

The activitity of the compounds of formula (I) towards preventing or treating the damage caused by a high level of atmospheric ozone was determined by comparing the skin of mice to which was applied the emulsion having the composition described in Example 1 above with that of mice not treated with this emulsion (control).

Ozone was produced from oxygen by electrical discharge using a Sander (Germany) model IV ozone generator. Once produced, the ozone was mixed with filtered ambient air (that is to say ozone-free air) and allowed to flow into a stainless-steel exposure chamber at a constant rate (200 1/minute). The ozone concentration in the exposure chamber was adjusted to 10 ppm and monitored using an ozone detector (Dasibi Model 1003-AH, CA, USA). The ozone chamber gave a maximum space to four mice for each exposure to ozone. The mice (4) shaved and sprinkled with the emulsion having the composition described in Example 1 above, were anaesthetized by intraperitoneal (i.p.) injection with sodium pentobarbital (50 mg/kg of body weight, sold by the company Abott Laboratories, IL, USA, under the name Nembutal) and left under anaesthesia throughout the period of exposure to the ozone (2 hours). The same experiment was then carried out on 4 mice not treated with the abovementioned emulsion (Control). At the end of the abovementioned exposure to ozone, the skin of the treated mice and that of the control mice was compared visually and it was seen that the skin of the mice sprinkled beforehand with the emulsion of the present invention showed no reddening, whereas that of the control mice showed extensive areas of reddening.

## Claims

1. Use of at least a compound of formula (I) 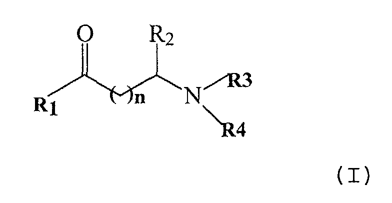 in which
n is 0 or 1,
R₁ is -OR₅ in which R₅ is hydrogen, linear or branched C1-C10 alkyl, C3-C10 cycloalkyl, C6-C10 aryl and C7-C10 arylalkyl or -NH-(CHR₆)-CH₂-(5-imidazole) in which R₆ is hydrogen or -COOR₅ in which R₅ has the meanings given above,
R₂ is chosen from the group comprising hydrogen, linear or branched C1-C10 alkyl, a group -R₇X in which R₇ is linear or branched, saturated or unsaturated C1-C18 alkyl, and X is a group chosen from imidazole, -N(R₈R'₈), -SR₈ in which R₈ and R'₈, which may be identical or different, are chosen from hydrogen, linear or branched C1-20 alkyl or alkenyl, C6-C10 aryl and C7-C10 arylalkyl; R₃ and R₄, which may be identical or different, are chosen from H, linear or branched C1-C10 alkyl, C3-C10 cycloalkyl, C6-C10 aryl, C7-C10 arylalkyl and alkylcarbonyl in which the C1-C20 alkyl is linear or branched and saturated or unsaturated; and
pharmaceutically acceptable salts thereof,
for the manufacture of a medicament for preventing or treating tissue damage caused by a high level of atmospheric ozone.

2. Use according to Claim 1, in which the said tissue damage is chosen from damage to skin tissues and to the tissues of the respiratory tract.

3. Use according to Claim 2, in which the damage to the tissues of the respiratory tract is an inflammation which leads to coughing, irritation of the nose and throat, a reduction in pulmonary function which is the cause of emphysema or bronchitis, sensitization to allergens leading to asthma attacks and reduction in the immune system, and the skin tissue damage is that leading to the formation of spots, dehydration, loss of elasticity, formation of wrinkles, reddening and loss of radiance.

4. Use according to any one of Claims 1 to 3, in which R₁ is chosen from -OH and -(CHR₆)-CH₂-(5-imidazole) in which R₆ is H, -COOH or -COOR₅ in which R₅ is chosen from methyl, ethyl, tert-butyl and octyl.

5. Use according to any one Claims 1 to 4, in which R₂ is chosen from the group comprising hydrogen, linear or branched C1-C6 alkyl, a group -R₇X in which R₇ is linear or branched, saturated or unsaturated C1-C10 alkyl, and X is a group chosen from imidazole, -N(R₈R'₈), -SR₈ in which R₈ and R'₈, which may be identical or different, are chosen from hydrogen, linear or branched C1-C10 alkyl or alkenyl, phenyl and benzyl.

6. Use according to any one of Claims 1 to 5, in which R₃ and R₄, which are different, are chosen from H, linear or branched C1-C4 alkyl, C5-C6 cycloalkyl, phenyl, benzyl and alkylcarbonyl in which the C1-C18 alkyl is linear or branched and saturated or unsaturated.

7. Use according to any one of Claims 1 to 6, in which the compounds of formula (I) are carnosine (compound of formula Ia), palmitoylcarnosinamide (compound of formula Ib), carnosine tert-butyl ester (compound of formula Ic), the dipeptide Ala-His (compound of formula Id), the dipeptide Lys-His (compound of formula Ie), lysine (compound of formula If), the dipeptide Ile-His (compound of formula Ig), decarboxycarnosine (compound of formula Ih), β-alanine (compound of formula Ii), histidine (compound of formula Il), the dipeptide Leu-His (compound of formula Im), the dipeptide Gly-His (compound of formula In), N-acetylcarnosine tert-butyl ester (compound of formula Io), the dipeptide Val-His (compound of formula Ip), N-acetylcysteine (compound of formula Iq), cysteine (compound of formula Ir), N-acetylcarnosine octyl ester (compound of formula Is) and N-acetylcarnosine (compound of formula It).

8. Compound of formula (I) 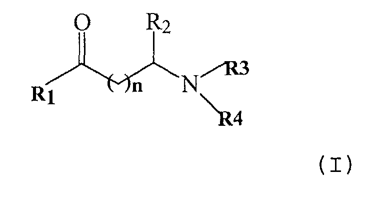 in which
n is 0 or 1,
R₁ is -OR₅ in which R₅ is hydrogen, linear or branched C1-C10 alkyl, C3-C10 cycloalkyl, C6-C10 aryl and C7-C10 arylalkyl or -NH-(CHR₆)-CH₂-(5-imidazole) in which R₆ is hydrogen or -COOR₅ in which R₅ has the meanings given above,
R₂ is chosen from the group comprising hydrogen, linear or branched C1-C10 alkyl, a group -R₇X in which R₇ is linear or branched, saturated or unsaturated C1-C18 alkyl, and X is a group chosen from imidazole, -N(R₈R'₈), -SR₈ in which R₈ and R'₈, which may be identical or different, are chosen from hydrogen, linear or branched C1-20 alkyl or alkenyl, C6-C10 aryl and C7-C10 arylalkyl; R₃ and R₄, which may be identical or different, are chosen from H, linear or branched C1-C10 alkyl, C3-C10 cycloalkyl, C6-C10 aryl, C7-C10 arylalkyl and alkylcarbonyl in which the C1-C20 alkyl is linear or branched and saturated or unsaturated; and
pharmaceutically acceptable salts thereof,
with the provision, however, that the said compound of formula (I) is not carnosine (compound of formula Ia), palmitoylcarnosinamide (compound of formula Ib), the dipeptide Ala-His (compound of formula Id), the dipeptide Lys-His (compound of formula Ie), the dipeptide Ile-His (compound of formula Ig), decarboxycarnosine (compound of formula Ih), β-alanine (compound of formula Ii), the dipeptide Leu-His (compound of formula Im), the dipeptide Gly-His (compound of formula In), the dipeptide Val-His (compound of formula Ip), N-acetylcarnosine (compound of formula It) or a compound of formula (I) in which, simultaneously, R₁ is OR₅, in which R₅ is hydrogen, and n is 0.

9. Compound according to Claim 8, in which the compound of formula (I) is chosen from carnosine tert-butyl ester (compound of formula Ic), N-acetylcarnosine tert-butyl ester (compound of formula Io) and N-acetylcarnosine octyl ester (compound of formula Is).

10. Compound as described in Claim 8 or 9, for use as a medicament.

11. Pharmaceutical composition comprising an effective amount of at least a compound of formula (I) as described in the preceeding Claim 8 or 9, together with at least a pharmaceutically acceptable inert excipient.

12. Pharmaceutical composition according to Claim 11, in the form of a cream, a nasal spray, a tablet, an aerosol or eye drops.

13. Pharmaceutical composition comprising an effective amount of at least a compound of formula (I) as described in the preceeding Claim 1 and at least an oxidizing agent together with at least a pharmaceutically acceptable inert excipient.

14. Composition according to Claim 13, in which the compound of formula (I) is that described in the preceeding Claim 8 or 9.

15. Pharmaceutical composition according to Claim 14, in which the antioxidant is chosen from extract of pomegranate and extract of green tea.

16. Pharmaceutical composition according to Claim 13, in which the compound of formula (I) is that described in the preceding Claim 7 and the antioxidant is chosen from extract of pomegranate and extract of green tea.

17. Cosmetic composition comprising at least a compound of formula (I) as described in the preceeding Claim 8 or 9, together with at least a cosmetically suitable inert excipient.

18. Cosmetic composition according to Claim 17, in the form of an emulsion, a balm, eye drops or a cream.

19. Cosmetic composition comprising an effective amount of at least a compound of formula (I) as described in the preceeding Claim 1 and at least an oxidizing agent together with at least a pharmaceutically acceptable inert excipient.

20. Cosmetic composition according to Claim 19, in which the compound of formula (I) is that described in the preceeding Claim 8 or 9.

21. Cosmetic composition according to Claim 20, in which the antioxidant is chosen from extract of pomegranate and extract of green tea.

22. Cosmetic composition according to Claim 19, in which the compound of formula (I) is that described in the preceeding Claim 7 and the antioxidant is chosen from extract of pomegranate and extract of green tea.

23. Use of at least a compound of formula (I) 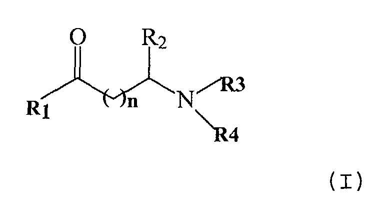 in which
n is 0 or 1,
R₁ is -OR₅ in which R₅ is hydrogen, linear or branched C1-C10 alkyl, C3-C10 cycloalkyl, C6-C10 aryl and C7-C10 arylalkyl or -NH-(CHR₆)-CH₂-(5-imidazole) in which R₆ is hydrogen or -COOR₅ in which R₅ has the meanings given above,
R₂ is chosen from the group comprising hydrogen, linear or branched C1-C10 alkyl, a group -R₇X in which R₇ is linear or branched, saturated or unsaturated C1-C18 alkyl, and X is a group chosen from imidazole, -N(R₈R'₈), -SR₈ in which R₈ and R'₈, which may be identical or different, are chosen from hydrogen, linear or branched C1-20 alkyl or alkenyl, C6-C10 aryl and C7-C10 arylalkyl; R₃ and R₄, which may be identical or different, are chosen from H, linear or branched C1-C10 alkyl, C3-C10 cycloalkyl, C6-C10 aryl, C7-C10 arylalkyl and alkylcarbonyl in which the C1-C20 alkyl is linear or branched and saturated or unsaturated; and
cosmetically suitable salts thereof,
as an anti-cutaneous-stress cosmetic agent.

24. Use according to Claim 23, in which the said anti-cutaneous-stress agent is non-oxidative.

25. Use according to Claim 23 or 24, in which the said cutaneous stress is that which leads to the formation of spots, dehydration, loss of elasticity, formation of wrinkles, reddening and loss of radiance.

26. Use according to any one of Claims 23 to 25, in which the compound of formula (I) is that described in the preceeding Claims 4, 5, 6 and 7.
